# EUROPEAN PATENT SPECIFICATION

(11) **EP 2 470 900 B1**
(45) Date of publication and mention of the grant of the patent: **03.12.2014**
(21) Application number: 10700071.3
(22) Date of filing: 12.01.2010
(51) Int. Cl.: G01N 33/50

(54) **A METHOD OF SCREENING AGENTS FOR THEIR IMPACT ON NUCLEIC ACID OXIDATION REACTIONS**
SCREENINGVERFAHREN FÜR WIRKSTOFFE AUF IHRE AUSWIRKUNG AUF NUKLEINSÄUREOXIDATIONSREAKTIONEN
PROCÉDÉ DE CRIBLAGE D'AGENTS SUR LA BASE DE LEUR EFFET SUR LES RÉACTIONS D'OXYDATION TOUCHANT DES ACIDES NUCLÉIQUES

(30) Priority: 25.08.2009 US 236666 P
(43) Date of publication of application: 04.07.2012
(73) Proprietor: Antec Leyden B.V., 2382 NV Zoeterwoude (NL)
(72) Inventor: OBERACHER, Herbert, A-6161 Natters (AT); PITTERL, Florian, A-6300 Wörgl (AT); CHERVET, Jean-Pierre, NL-1017 CC Amsterdam (NL)
(74) Representative: Nederlandsch Octrooibureau
(86) International application number: PCT/NL2010/050014
(87) International publication number: WO 2011/025366

(56) References cited:
- WO-A2-03/081208
- DENG HAITENG ET AL: "A thin-layer electrochemical flow cell coupled on-line with electrospray-mass spectrometry for the study of biological redox reactions" ELECTROANALYSIS, vol. 11, no. 12, August 1999 (1999-08), pages 857-865, XP002577513 ISSN: 1040-0397 cited in the application
- JURVA ULRIK ET AL: "Comparison between electrochemistry/mass spectrometry and cytochrome P450 catalyzed oxidation reactions." RAPID COMMUNICATIONS IN MASS SPECTROMETRY, vol. 17, no. 8, 2003, pages 800-810, XP002577514 ISSN: 0951-4198 cited in the application
- GAMACHE P H ET AL: "Metabolomic applications of electrochemistry/Mass spectrometry" JOURNAL OF THE AMERICAN SOCIETY FOR MASS SPECTROMETRY, ELSEVIER SCIENCE INC, US LNKD- DOI:10.1016/J.JASMS.2004.08.016, vol. 15, no. 12, 1 December 2004 (2004-12-01), pages 1717-1726, XP004678510 ISSN: 1044-0305
- FARMER P B ET AL: "Use of DNA adducts to identify human health risk from exposure to hazardous environmental pollutants: The increasing role of mass spectrometry in assessing biologically effective doses of genotoxic carcinogens" MUTATION RESEARCH. REVIEWS IN MUTATION RESEARCH, ELSEVIER, AMSTERDAM, NL LNKD- DOI:10.1016/J.MRREV.2008.03.006, vol. 659, no. 1-2, 1 July 2008 (2008-07-01), pages 68-76, XP023315383 ISSN: 1383-5742 [retrieved on 2008-04-08]
- BAUMANN ANNE ET AL: "On-Line Electrochemistry/Electrospray Ionization Mass Spectrometry (EC/ESI-MS) for the Generation and Identification of Nucleotide Oxidation Products" ELECTROANALYSIS, vol. 22, no. 3, 27 November 2009 (2009-11-27), pages 286-292, XP002577515 ISSN: 1040-0397
- SANDRA JAHN ET AL: "Electrochemistry coupled to (liquid chromatography/) mass spectrometry-Current state and future perspectives", JOURNAL OF CHROMATOGRAPHY A, vol. 1259, 1 October 2012 (2012-10-01), pages 16-49, XP055118361, ISSN: 0021-9673, DOI: 10.1016/j.chroma.2012.05.066

## Description

### TECHNICAL FIELD OF THE INVENTION

The present invention relates to a method of determining the impact of agents on oxidation reactions of nucleic acid species selected from nucleosides, nucleotides, oligonucleotides, polynucleotides and combinations thereof. More particularly, the present invention provides a highly sensitive screening method for determining the effect of all kinds of agents (e.g. drugs, nutrients, contaminants) on oxidation reactions involving the aforementioned nucleic acid species, said method using electrochemical oxidation of the nucleic acid species followed by on-line determination of the oxidation profile of the oxidized sample with the help of Electrospray-Mass Spectrometry (ESI-MS) or Matrix Assisted Laser Desorption Ionization-Mass Spectrometry (MALDI-MS).

The present method enables identification of agents that are capable of promoting or preventing *in vivo* nucleic acid oxidation or that are capable of participating in *in vivo* reactions that lead to the formation of adducts of these agents and oxidation products of nucleic acid species.

### BACKGROUND OF THE INVENTION

Nucleic acids present within living systems are continuously exposed to reactive chemicals. The resulting modification of nucleic acids causes molecular disturbance of the genetic material. Modified purine and pyrimidine bases are potential substrates for repair enzymes or polymerases, or they can block these activities, triggering biological responses including mutation, cell death, malignancy, and aging.

One class of reactive chemicals that can induce modification of nucleic acids are reactive oxygen species (ROS). Major efforts have been devoted during the last two decades to elucidate the oxidation pathways mediated by'^{O}₂, OH, and one-electron oxidants inducing "oxidative damage" of genetic material. More than 100 different nucleotides resulting from reaction with ROS have been isolated and characterized which clearly indicates the complexity and diversity of nucleic acid oxidation reactions.

Electrochemistry (EC) is an established method for studying redox reactions of biomolecules, including nucleic acids. The electroactivity of nucleic acids was discovered in the 1960s. Oxidation mainly relies on the intrinsic electrochemical activity of the nucleobases, even though the oxidation of the sugars has also been demonstrated.

EC can be applied for the detection of nucleic acids species. Due to its low limits of detection EC is often part of liquid chromatographic methods developed for the quantification of nucleobases, nucleosides or nucleotides. Modified species have been detected in vivo in the range 1/10¹¹ nucleotides (which might be found in human tissues following administration of single doses of genotoxic compounds) to 1/10⁴ nucleotides (which may be formed in experimental systems after chronic treatment with a carcinogenic dose of a compound) enabling the investigation of various enogenous and exogeneous sources of hazard on genetic material.

EC has been extensively applied to study the redox properties of nucleic acids to get a better understanding of biologically relevant processes. Particularly, cyclic voltammetry on different types of electrode materials was used to estimate oxidation potentials and to study oxidation mechanisms. After isolation, products obtained upon electrochemical oxidation of nucleobases, nucleosides, and mononucleotides were characterized with different techniques including UV/VIS spectroscopy and gas chromatography-mass spectrometry after derivatization. These experiments revealed that guanine oxidation occurs in an overall 4H⁺, 4e⁻ process. Major oxidation products include dimeric guanine, 8-hydroxyguanine as well as degradation products including 5-hydroxyhydantoin-5-carboxamid and monohydrated alloxan.

On-line EC/thermospray-MS for studying non-volatile compounds in solution was introduced by Hambitzer and Heitbaum in 1986 *(*Anal Chem 1986, 58, 1067-1070). They constructed a three electrode assembly for electrochemical oxidation of N,N-dimethylaniline and connected it with thermospray-MS.

The oxidation pathway of uric acid, was investigated by Brajther-Toth and co-workers *(*Anal Chem 1988, 60, 720-2; Anal Chem 1989, 61, 1709-17; and Anal Chem 1992, 64, 21A-33A.). EC/MS was established with both thermospray and ESI. Dimeric uric acid as well as allantoin, alloxan, and parabenic acid were identified as final products of electrochemical oxidation.

The first paper demonstrating the usability of electrospray ionization (ESI) to monitor electrochemical oxidation processes was published by Deng and Van Berkel (Electroanalysis 1999, 11, 857-65). They coupled a three electrode thin-layer, flow-by electrode cell with ESI-MS to study the oxidation of dopamine.

Bruins and co-workers have systematically investigated oxidation reactions that can induced with EC (Rapid Commun Mass Spectrom 2000, 14, 529-33 and Rapid Commun Mass Spectrom 2003, 17, 800-10). They concluded that mainly 1e⁻ oxidation reactions, such as N-dealkylation, S-oxidation, P-oxidation, alcohol oxidation and dehydrogenation, can successfully be mimicked to study the oxidative metabolism of pesticides and drugs, including caffeic acid, diclofenac, troglitazone, tetrazepam, and toremifen.

Bruins and co-workers *(*Rapid Commun Mass Spectrom 2003, 17, 1585-92 and J Am Soc Mass Spectrom 2004, 15, 1707-16) have further introduced EC as an alternative protein digestion method and were able to cleave proteins, including insulin, α-lactalbumin and carbonic anhydrase, at tyrosine and tryptophan residues. Girault and co-workers (Chemphyschem 2003, 4, 200-6 and J Proteome Res 2006, 5, 793-800) studied electrochemically induced tagging of cysteine residues in peptides and proteins to probe their environment. EC/ESI-MS has also been used to simulate oxidative metabolism.

Despite the fact that oxidation of nucleic acid species is a widely researched area, progress in this area has been hindered by the fact that there does not exist a fast, sensitive and reliable screening tool for studying these oxidation reactions. This is not surprising given that these oxidation processes occur within very complex biological systems, generating very low concentrations of a wide variety of oxidation products.

### SUMMARY OF THE INVENTION

The inventors have developed a method that enables fast screening of all kinds of agents for their impact on the oxidation of nucleic acids.

The present method is not only fast, but it is also extremely sensitive and it produces results that are highly predictive of the effect these same agents have on *in vivo* oxidation of nucleic acid species.

The present invention provides a method of determining the impact of an agent on oxidation reactions of a nucleic acid species, said method comprising the steps of:
- providing a reference sample containing a nucleic acid species;
- providing a test sample containing the same nucleic acid species as the reference sample;
- oxidizing the reference sample and the test sample by submitting each sample to identical electrochemical oxidation conditions;
- on-line determining the oxidation profile of the electrochemically oxidized reference sample and of the electrochemically oxidized test sample using Electrospray Ionization-Mass Spectrometry (ESI-MS) or Matrix Assisted Laser Desorption Ionization-Mass Spectrometry (MALDI-MS); and
- determining the impact of the agent on oxidation reactions of nucleic acid species by comparing the oxidation profiles of the reference sample and the test sample
wherein the agent is added to the test sample before the on-line determining of the oxidation profile.

The present method makes it possible to avoid the time-consuming isolation and derivatization steps that are frequently employed in scientific investigations of nucleic acid oxidation processes. In addition, because the present method is highly predictive of the effect of agents on *in vivo* oxidation of nucleic acid species, the present method reduces the need for *in vitro* and, more importantly, *in vivo* studies.

The present screening method may advantageously be employed to identify agents that potentially have *in vivo* anti-oxidative potency and that may be employed to diminish undesirable DNA/RNA oxidation processes in humans or animals. Likewise, the screening method may be employed to evaluate the potential effect of known drugs or nutrients on DNA/RNA oxidation processes.

The present method can also be used to identify agents that are capable of reacting with oxidation products of nucleic acid species to form adducts. Identification of agents having this capability is important because these agents might induce diverse biological effects due to their mutagenic, genotoxic, or carcinogenic properties.

The data collected by the inventors clearly demonstrate that the electrochemical oxidation of the nucleotide species performed within the present method can closely mimic *in vivo* oxidation processes involved in the alteration of nucleic acids, such as nucleosides, nucleotides and small oligomers. The hyphenation of electrochemical oxidation to ESI-MS detection enables online monitoring as well as structure elucidation of the reaction products.

### FIGURES

- Figure 1:: EC/ESI-MS spectrum of guanosine. Mass spectra were measured
(a) in positive ion mode (E = 2.25 V) and
(b) in negative ion mode (E = 2.0 V).
- Figure 2:: Tandem mass spectra of the electrochemically produced guanosine dimer.
(a) Fragmentation of the protonated dimer (E = 2.25 V, CE = 15 eV).
(b) Fragmentation of the deprotonated dimer (E = 2.0 V, CE = -10 eV).
- Figure 3:: Tandem mass spectra of singly oxidized guanosine.
(a) Fragmentation of the protonated nucleobase (E = 2.25 V, CE = 10 eV).
(b) Fragmentation of the deprotonated nucleobase (E = 2.0 V, CE = -5 eV).
(c) Tandem mass spectrum of the protonated nucleobase cleaved from singly oxidized guanosine via in-source collision induced dissociation (E = 2.0 V, CE = 15 eV).

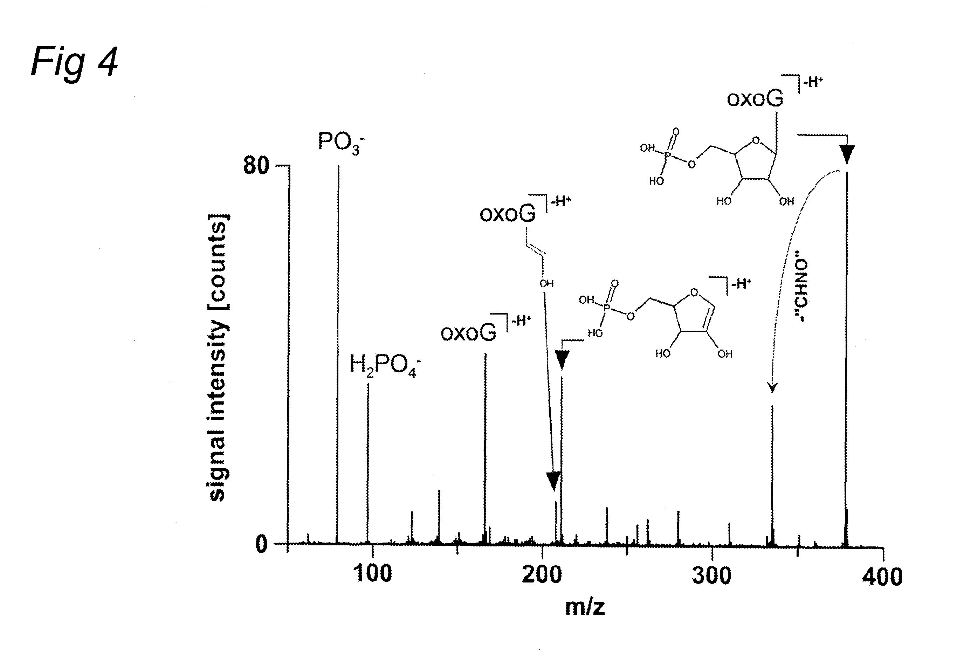

- Figure 4:: Tandem mass spectrum of singly oxidized GMP (E = 2.0 V, CE = -15 eV).

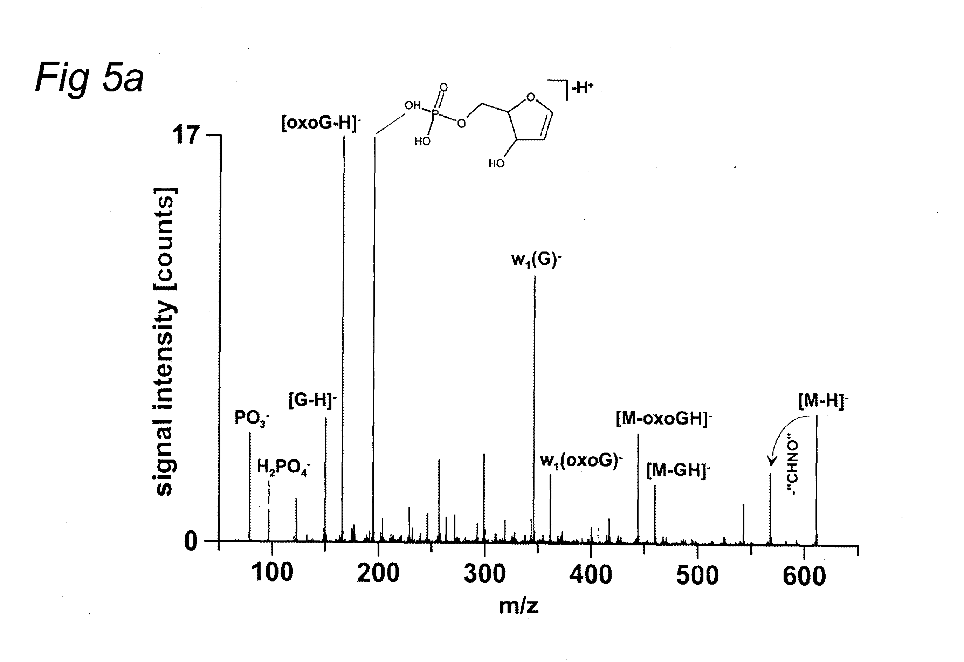

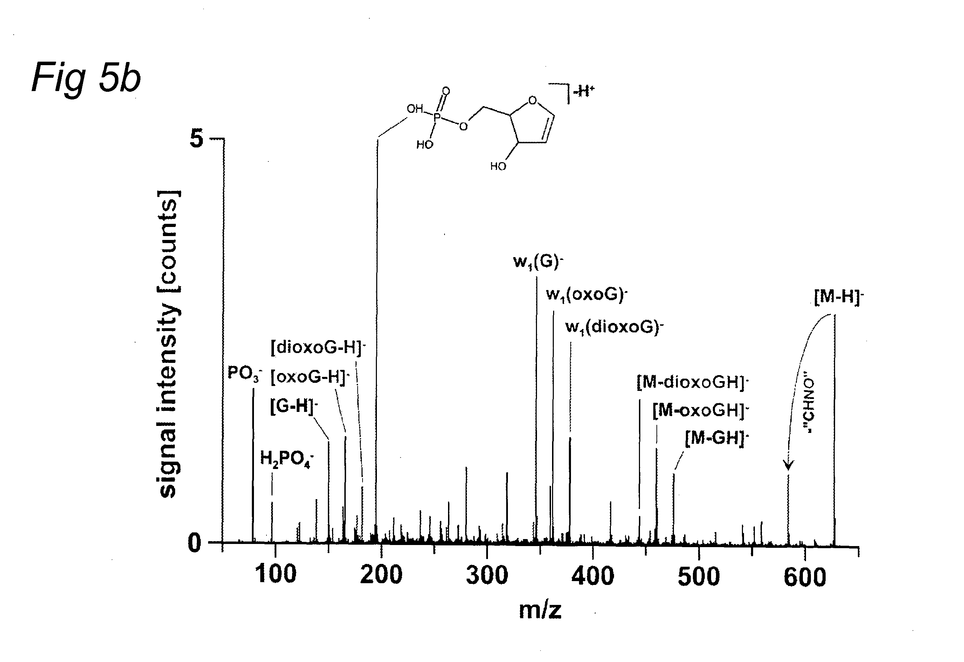

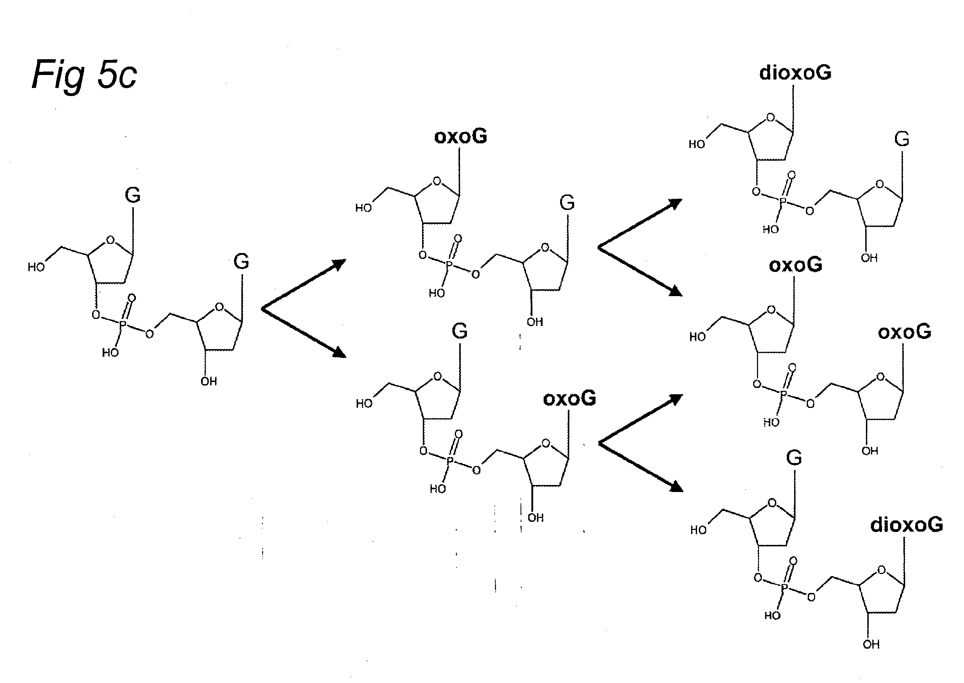

- Figure 5:: Tandem mass spectra of:
(a) the singly oxidized forms (E = 1.5 V, CE = -40 eV) and
(b) the doubly oxidized forms of dGpdG (E = 1.5 V, CE = -40 eV).
(c) Formation of different doubly oxidized forms of dGpdG via electrochemical oxidation.

### DETAILED DESCRIPTION OF THE INVENTION

Accordingly, the present invention provides a method of determining the impact of an agent on oxidation reactions of a nucleic acid species selected from nucleosides, nucleotides, oligonucleotides, polynucleotides and combinations thereof, said method comprising the steps of:
- providing a reference sample containing a nucleic acid species;
- providing a test sample containing the same nucleic acid species as the reference sample;
- oxidizing the reference sample and the test sample by submitting each sample to identical electrochemical oxidation conditions, said oxidation conditions being sufficiently severe to produce detectable levels of oxidation products of the nucleic species in the reference sample;
- on-line determining the oxidation profile of the electrochemically oxidized reference sample and of the electrochemically oxidized test sample using Electrospray Ionization Mass Spectrometry (ESI-MS) or Matrix Assisted Laser Desorption Ionization-Mass Spectrometry (MALDI-MS); and
- determining the impact of the agent on oxidation reactions of nucleic acid species by comparing the oxidation profiles of the reference sample and the test sample;
wherein the agent is added to the test sample before the on-line determining of the oxidation profile.

The term "oligonucleotide" as used herein refers to a deoxyribonucleic acid (DNA) or ribonucleic acid (RNA) sequence composed of 2-100 covalently linked nucleotides.

The term "polynucleotide" as used herein refers to a deoxyribonucleic acid (DNA) or ribonucleic acid (RNA) sequence composed of more than 100 covalently linked nucleotides.

The term "oxidation" as used herein refers to the loss of electrons / hydrogen or the gain of oxygen / increase in oxidation state by a molecule, atom or ion.

The terminology "electrochemical oxidation" as used herein refers to an oxidation reaction that occurs in, or is initiated in a solution at the interface of an electron conductor (an electrode) and an ionic conductor (the electrolyte), and which is driven by an external applied voltage. In the course of electrochemical oxidation nucleic acids species can be oxidized directly or indirectly. During indirect oxidation the nucleic acid species is oxidized through a reaction with an electrochemically oxidized precursor (e.g. a hydroxyl radical or Ru complexes).The term "detectable levels of oxidation products" as used herein means that the electrochemical formation of oxidation products of the nucleic acid species is evident in the oxidation profile.

The term "mass spectrometry" as used herein also encompasses tandem mass spectrometry (MS/MS).

In the present method the agent to be tested is added to the test sample before the on-line determining of the oxidation profile. The agent can be added before, during or after the test sample is subjected to electrochemical oxidation conditions. Typically, the agent is added at least 2 seconds before the on-line determining of the oxidation profile in order to allow sufficient time for the agent to interact with oxidation products of the nucleic species.

The oxidation of the reference sample and the test sample may suitably occur in an electrochemical cell. The electrochemical cell may be coupled directly to the ESI-MS or MALDI-MS, but the invention also encompasses configurations in which the oxidized samples are subjected to one or more additional analytical procedures (e.g. extraction, chromatography, electrophoresis), before being fed to the ESI-MS or the MALDI-MS.

The comparison of the oxidation profiles of the reference sample and the test sample in the present method aims to identify differences between these oxidation profiles that are associated with the presence of the agent in the test sample.

Advantageously, the comparison of the oxidation profiles of the reference sample and the test sample comprises quantitative comparison of the recorded intensity at one or more predetermined mass-to-charge (m/z) ratios for each sample, said m/z ratios coinciding with the m/z ratios of one or more oxidation products of nucleic acid species (e.g. biomarkers). The use of predetermined m/z ratios for the comparison of the oxidation profiles makes it possible to automate this comparison operation, and to produce the results of the comparison on-line.

In accordance with another advantageous embodiment, the comparison of the oxidation profiles of the reference sample and the test sample comprises identifying m/z ratios having a statistically significant higher intensity in the oxidation profile of the test sample than in the oxidation profile of the reference sample and determining whether these m/z ratios are associated with a potential adduct of the agent and an oxidation product of the nucleic acid species. The present method enables the fast detection of potential adducts at very low concentration levels.

The reference sample and the test sample employed in the present method advantageously contain a solvent, preferably a solvent selected from water, organic solvents and combinations thereof. Preferably, solvent is contained in these samples in a sufficient amount to render these samples liquid. Typically, the reference sample and the test sample contain at least 90 wt.% of solvent, most preferably at least 95 wt.% of solvent. The water content of the reference sample and test sample advantageously exceeds 20 wt.%, most preferably it exceeds 40 wt.%.

According to another particularly preferred embodiment of the invention, the on-line determining of the oxidation profile comprises feeding at least a part of the electrochemically oxidized reference sample and at least a part of the electrochemically oxidized test sample to a chemical separation device that is coupled to the ESI-MS or the MALDI-MS, using identical separation conditions in the separation device for the oxidized samples. Examples of chemical separation devices that may be employed included a liquid chromatograph (including HPLC and UPLC), a solid-phase extraction device and an electrophoresis device. Preferably, the method employs a chemical separation device in the form of a liquid chromatograph or an electrophoresis device. Even more preferably, the method employs a liquid chromatograph, most preferably an HPLC or an UPLC.

The use of a chemical separation device has the drawback that the determination of the oxidation profile takes more time than in case the oxidized samples are immediately fed to the ESI MS or MALDI-MS. However, the introduction of a chemical separation device, such as HPLC, offers the very important advantage that the sensitivity and especially the reliability of the present method can be increased substantially, especially in case samples of a complex nature are analyzed with the present method.

According to a further preferred embodiment of the present method both the reference sample and the test sample are oxidized in a Flow-through Electrochemical Cell (FEC) that is advantageously coupled to the chemical separation device, said electrochemical cell having a working volume of 1.0 nl to 1.0 ml and being operated at a flow rate of 1.0 nl/min. to 10 ml/min. The use of an FEC offers the advantage that very small sample volumes can be handled and that 'carry-over' effects can be minimized very effectively. Particularly good results can be obtained with the present method using an FEC having a working volume of 5.0 nl to 100 µl, more preferably of 10 nl to 30 µl, and most preferably of 30 nl to 10 µl.

The sample flow rate employed in the FEC preferably is within the range of 10 nl/min. to 3.0 ml/min. More preferably, said flow rate lies within the range of 30 nl/min. to 1.0 ml/min. and most preferably the FEC is operated at a flow rate of 100 nl/min. to 300 µl/min.

As explained herein before, the agent to be tested in the present method can be added before, during or after the test sample is subjected to electrochemical oxidation conditions. Preferably, the agent is added at least 5 seconds, more preferably 10-100 seconds before the on-line determining of the oxidation profile.

According to a preferred embodiment, the agent is added to the test sample before or during the electrochemical oxidation. Most preferably, the agent is added to the test sample before the sample is subjected to the electrochemical oxidation conditions. Having the agent present in the test sample during the electrochemical oxidation offers the advantage that the method may be used to elucidate the effect of the agent on the oxidation (oxidation pathways, oxidation rates etc.) of the nucleic acid species. Furthermore, if during the electrochemical oxidation the agent is oxidized, this embodiment enables the study of reactions occurring between the oxidized agent and the oxidized and non-oxidized nucleic acid species.

According to another preferred embodiment, the agent is added to the test sample after the sample has been subjected to the electrochemical oxidation conditions. This embodiment offers the advantage that it is very suitable for elucidating reactions that occur between the agent and oxidation products of the nucleic acid species. Especially if during the electrochemical oxidation the agent would have been oxidized, this embodiment enables the study of reactions occurring between the non-oxidized agent and the oxidized nucleic acid species.

In order to maximize sensitivity it is preferred to collect at least 1.0 nl of oxidized eluate from the FEC and to subsequently introduce the collected sample into the chemical separation device for on-line determining of the oxidation profile. In case the oxidized samples are produced within a time frame of a few seconds, the sample collection step could be avoided. However, even in these cases sample collection is preferred as it yields a better signal to noise (S/N) ratio.

In the present method sample collection typically takes 0.1 seconds to 100 minutes, more preferably 1 seconds to 10 minutes, and most preferably 10 seconds to 1 minute. Sample collection in accordance with the presence method may suitably occur in e.g. a transfer line or an injection loop.

Advantageously 10 nl to 500 µl of oxidized eluate is collected before being introduced into the chemical separation device for on-line determining of the oxidation profile. Even more preferably, the amount of oxidize eluate that is collected is within the range of 50 nl to 300 µl, most preferably within the range of 100 nl to 200 µl.

The oxidation of the reference sample and test sample can suitably be conducted in different electrochemical cells, provided these cells are operated under exactly the -same conditions. Alternatively, the reference sample and the test sample can be oxidized in the same electrochemical cell. By oxidizing the samples in the same electrochemical cell it can be ensured that the oxidation conditions employed for the test samples and the reference sample-are as close as possible. Furthermore, the reference sample can suitably be used to flush the electrochemical cell after the electrochemical oxidation of each test sample.

In order to achieve high conversion rates in the FEC, it is advantageous to employ a cell in which the working electrode defines at least 10 % of the inner surface area of the working volume. Even more preferably, the working electrode defines at least 20% of the inner surface of the working volume, most preferably it defines at least 25% of the inner surface of the working volume. Usually, the working electrode defines not more than 60% of the inner surface of the working volume.

The FEC employed in the present method typically comprises a working electrode and an auxiliary electrode, the distance between the working electrode and the auxiliary electrode being in the range of 1 µm to 1 cm, more preferably in the range of 10 µm to 1 mm, and most preferably in the range of 30 µm to 300 µm.

According to a preferred embodiment, the oxidation of the samples is performed in an electrochemical cell comprising a working electrode, an auxiliary electrode and a reference electrode. Although electrochemical oxidation of the samples can be achieved using a two-electrode system that comprises a working electrode and an auxiliary electrode, the use of a three-electrode system that additionally contains a reference electrode, offers the advantage that changes in working potential due to polarization effects can be avoided.

Examples of working electrodes that may suitably be employed in the present method include a conductive diamond working electrode, a platinum working electrode, a glassy carbon working electrode and a golden working electrode. According to a particularly preferred embodiment, the working electrode is a conductive diamond working electrode, preferably a boron doped diamond electrode or a diamond coated glassy carbon electrode.

The present method is particularly suited for screening samples of very complex composition, notably samples that contain biological material. Accordingly, in a preferred embodiment of the present method the same biological material is present in the reference sample and the test sample, said biological material being selected from a bodily fluid, tissue or a material derived from bodily fluid or tissue (e.g. cells or cell organelles). The biological material may provide a part or all of the nucleic acid species present in the samples. Typically, the biological material represents at least 0.0001 wt.%, more preferably at least 0.1 wt.% of the solute contained in the reference sample.

In the present method the samples are preferably oxidized by employing a voltage in the range of 100-5000 mV. Even more preferably, the voltage employed is in the range of 200-4500mV, most preferably in the range of 400-4000 mV.

Typically, in the present method the samples are subjected to electrochemical oxidation conditions for 0.01-1000 seconds, more particularly 0.1-100 seconds. Most preferably, the samples are subjected to electrochemical oxidation conditions for 1-10 seconds.

In order to allow for e.g high throughput screening, the test samples are advantageously provided by an autosampler. In accordance with this preferred embodiment an autosampler may be used to transfer test samples to the FEC. The autosampler may also be used to provide the reference sample, but this is not necessary.

The use of an autosampler enables automatic on-line determining of the oxidation profiles of a large number of test samples. Accordingly, in accordance with a particularly preferred embodiment, the method comprises on-line determining the oxidation profiles of at least 10 test samples, more preferably of at least 20 test samples and most preferably of at least 96 test samples in a single run of the autosampler. Here a single run refers to a single operation of the autosampler in which a plurality of samples are processed consecutively in a preprogrammed fashion.

Even if the present method employs a chemical separation device, the present method can suitably be used for on-line determining of at least 2 oxidation profiles of electrochemically oxidized samples per hour.

In case no chemical separation device is used, on-line determining of at least 10 oxidation profiles is perfectly feasible. Thus, the present method may comprise on-line determining of at least 10 oxidation profiles of electrochemically oxidized samples per hour by directly transferring the oxidized samples to the ESI-MS or the MALDI-MS without prior chromatographic separation.

The present method is particularly suitable for screening the anti-oxidative or pro-oxidative effects of agents on nucleic acid species selected from nucleotides, oligonucleotides and combinations thereof. The present method is particularly-suited for screening such effects on oligonucleotides.

The samples that can be analyzed in the present method may contain nucleic acid species within a very wide concentration range as the present method can suitably be used to analyze samples containing no more than traces of nucleic acid species as well as samples of isolated nucleic acid species. Consequently, the samples used in the present method suitably contain 1 femtoM to 100 M of the nucleic acid species. Preferably, the samples contain 1 picoM to 0.001 M of the nucleic acid species.

As explained herein before, the present method offers the advantage that it does not require any chemical derivatization. Consequently, in a preferred embodiment, the method does not employ derivatization of the oxidation products formed from the nucleic acid species during the electrochemical oxidation.

The invention is further illustrated by the following non-limiting examples.

### EXAMPLES

### Example 1

### EC/ESI-MS of nucleosides

The oxidative behavior of guanosine, adenosine, cytidine and uridine were studied with on-line EC/ESI-MS.

The following sample solutions were used for electrochemical conversion:
(a) 100 µM guanosine in 10 mM ammonium formate (pH 7.3) containing 50% acetonitrile (v/v);
(b) 100 µM adenosine in 10 mM ammonium formate (pH 7.3) containing 50% acetonitrile (v/v);
(c) 100 µM cytidine in 10 mM ammonium formate (pH 7.3) containing 50% acetonitrile (v/v);
(d) 100 µM uridine in 10 mM ammonium formate (pH 7.3) containing 50% acetonitrile (v/v).

Electrochemical simulations were performed in an electrochemical wall-jet cell (Flex Cell®, Antec Leyden, Zoeterwoude, The Netherlands). A conductive diamond electrode (Magic Diamond®, Antec Leyden) was used as working electrode material. Due to its chemical inertness, high electrical conductivity, extraordinarily low catalytic activity for both hydrogen and oxygen evolution and very low background current within a wide working potential range, conductive diamond is an ideal electrode material for the oxidation of nucleic acids. A platinum electrode was used as counter electrode. The HyREF® (Antec Leyden) electrode was used as reference electrode.

Potentials (0.0 - 4.0 V) were applied using the electrochemical detector system Decade II (Antec Leyden). Sample solutions were passed through the cell by a syringe pump at a flow rate of 3.0 µL/min. The cell was coupled directly to the mass spectrometer (Qstar® XL, Applied Biosystems, Foster City, CA, USA). A grounded stainless steel union was used to decouple the electrospray potential from the potential applied at the electrochemical cell. ESI was performed with a modified TurboIonSpray source (transfer line: 375 µm o.d., 50 µm i.d., sprayer capillary: 90 µm o.d., 20 µm i.d., Polymicro Technologies Phoenix, AZ, USA).

Mass calibration was performed in positive mode by infusion of a mixture of 1.0 mg/l caffeine and 1.0 mg/l reserpine dissolved in 0.05% aqueous acetic acid solution containing 50% acetonitrile (v/v) at a flow rate of 3.0 µl/min. Mass spectrometric parameters for both ionisation modes were optimized using a 100 µM solution of guanosine in 10 mM ammonium formate (pH 7.3) containing 50% acetonitrile (v/v).

Electrochemical conversions were monitored in both ionisation modes. In positive ion mode the spray voltage was set to 4.5 kV. In negative ion mode a spray voltage of -4.0 kV was applied. Gas flows of 2-5 arbitrary units (nebulizer gas) and 20-25 arbitrary units (turbo gas) were employed. The temperature of the turbo gas was adjusted to 200 °C. The scan range was 100-600 u. The accumulation time was set to 1.0 sec. For MS/MS, the Q1 resolution was set to unit resolution. The collision gas (N2) flow was set to 5 arbitrary units. Applied collision voltages depended on the fragmentation behavior of the investigated molecules. Total ion chromatograms and mass spectra were recorded on a personal computer with the Analyst QS software (1.0, service pack 8, Applied Biosystems).

Sample solutions were sprayed from a capillary that was connected to the high-voltage end of the power supply. A potentiostat was used to control the working electrode potential in the three-electrode configuration. The transformations of guanosine and adenine were monitored in positive and negative ionization mode. Cytidine and uridine oxidation were studied in negative ion mode. For each nucleoside the signal intensity was extracted out of the mass spectrometric data and plotted against the potential set at the potentiostate (E). It was found that in positive ion mode oxidation of guanosine started at 1500 mV. Interestingly, in negative ion mode guanosine was already oxidized at 500 mV. The discrepancy clearly suggests that the high voltage of the ESI source has some impact on the potential applied at the working electrode. This hypothesis was supported by the results obtained for adenosine.

The potential applied at the potentiostat necessary to induce adenosine oxidation was shifted from 2500 mV to 1250 mV by switching from positive to negative mode. A clear advantage of the coupled potentials was the possibility to initiate oxidation of cytidine and uridine at reasonable potentials. For both species oxidation was monitored in negative ionization mode, thus shifting the potential necessary to nearly completely decompose the nucleosides below 4000 mV. As the potential of the potentiostate does not represent the actual potential at the working electrode given values need to be interpreted with caution. Nevertheless, the observed order (guanosine<<adenosine<cytidine<uridine) is in full concordance with published data (Oliveira-Brett et al. Anal Biochem 2004, 332, 321-9; Seidel et al. J. Phys. Chem. 1996, 100, 5541-53).

### Example 2

### Electrochemical oxidation of guanosine

The first to oxidize the base of nucleic acids is guanine. Thus, its oxidation has been extensively studied in the context of DNA damage, associated with mutation and aging. A large number of oxidative lesions that arise from the oxidation of guanine under a variety of conditions have been described in literature. Accordingly, of all nucleosides guanosine is the ideal model compound for evaluating the usefulness of on-line EC/ESI-MS to mimic oxidation processes involving nucleic acids.

The following sample solution was used for electrochemical conversion:
100 µM guanosine in 10 mM ammonium formate (pH 7.3) containing 50% acetonitrile (v/v).

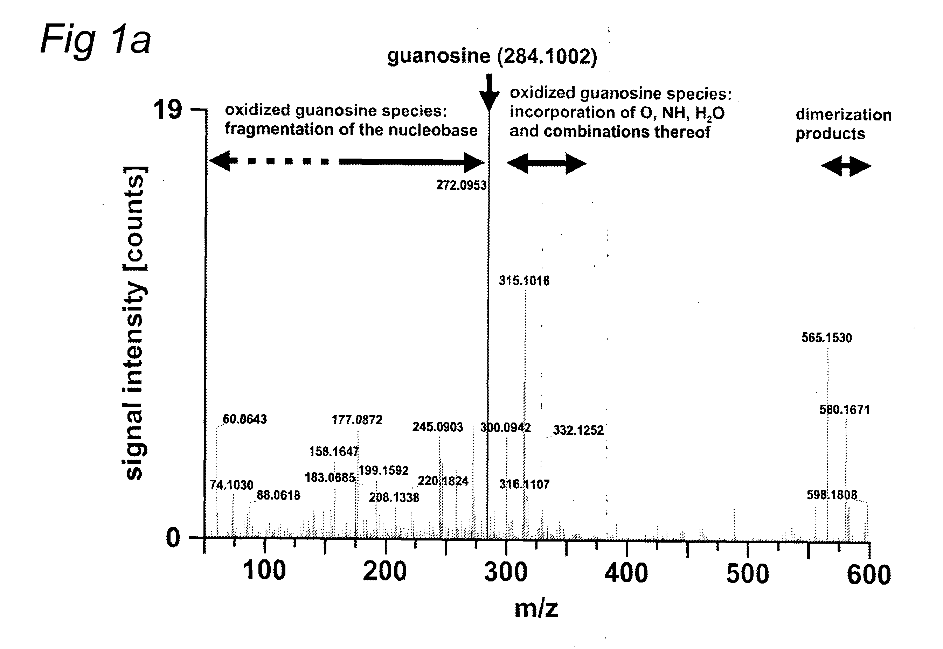

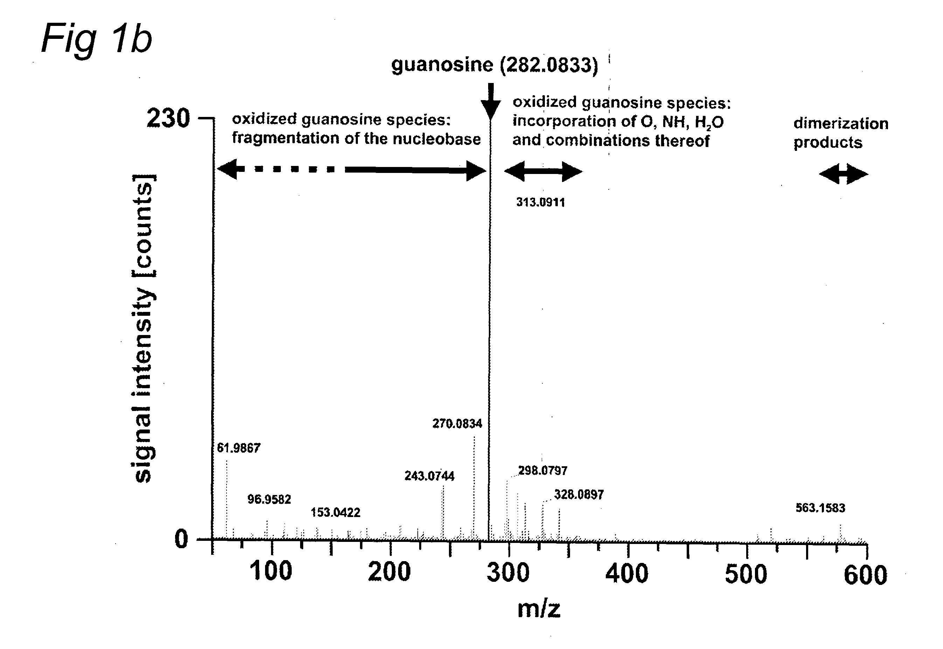

The sample solution was passed through the electrochemical cell and monitored with ESI-MS in both ionization modes. Representative mass spectra are depicted in Figure 1. Background subtraction was made by subtracting mass spectra recorded at zero potential to facilitate the detection of oxidation products. Subtracted spectra appeared to contain a large number of signals indicating the complexity of nucleic acids oxidation and the diversity of possible reaction pathways. Species showing an increase of signal intensity with increasing potential were considered as tentative oxidation products of guanosine. Irrespective of the ionization mode used to monitor oxidation reactions, similar compounds exhibited increased signal intensities. Due to differences in the ionization efficiencies relative intensities varied. Measured molecular masses were used to determine molecular formulas and thus to predict putative transformation reactions. The deviation between the measured and the calculated molecular mass was typically far below 50 ppm.

Three different groups of oxidation products were identified. One group resulted from the addition of [O], [NH], water or combinations thereof to guanosine. The incorporation of up to four different groups was observed indicating the occurrence of 1-4 e⁻ reactions. The most abundant signals corresponded to monooxidized guanosine (C₁₀H₁₃N₅O₆), "guanosine + HNO" (C₁₀H₁₄N₆O₆), and the doubly oxidized guanosine (C₁₀H₁₃N₅O₇). These species are well documented products of guanine oxidation, in particular with electrochemical methods. Among those the monooxidized form (8-hydroxyguanine) represents the overall most well-studied DNA oxidation lesion and is often employed as biomarker of oxidative stress.

Another important group of oxidation products resulted from secondary oxidation processes leading to the decomposition of the nucleobase. One prominent member of this group was a species being 12.001 u (C₉H₁₃N₅O₅) lighter than guanosine. Several different structures corresponding to the determined molecular formula have been found in literature. Another abundant species was 39.010 u (C₈H₁₁N₄O₅) lighter than guanosine and could represent 2-amino-5-[(β-D-erythropentofuranosyl)amino]-4H-imidazol-4-one. The imidazolone represents a well-studied primary oxidation product of 2'-deoxyguanosine.

The third group of oxidation products included dimeric species. The most abundant signal was assigned to (guanosine-H)₂ (C₂₀H₁₁N₁₀O₁₀). Additionally, several species originating from the dimerization of oxidized species were observed. Guanine oligomerization during electrochemical oxidation of guanosine has also been reported in literature.

Accurate molecular mass measurements enable the prediction of molecular formulas of oxidation products, and thus give valuable information on the kind of transformation reaction that might have occurred. However, such data does not provide any information on the site of modification. In an attempt to elucidate the structure of guanosine oxidation products, tandem mass spectral data was acquired and interpreted. A limited number of fragmentation pathways were detected. Irrespective of the ionization mode applied, the most commonly observed fragmentation reaction upon collision-induced decomposition of guanosine derivates was the cleavage of the C-N bond between the sugar and the nucleobase giving rise to intense signals of the nucleobase. The corresponding neutral loss was used as an unequivocal identifier for the structural relatedness of a putative oxidation product to guanosine.

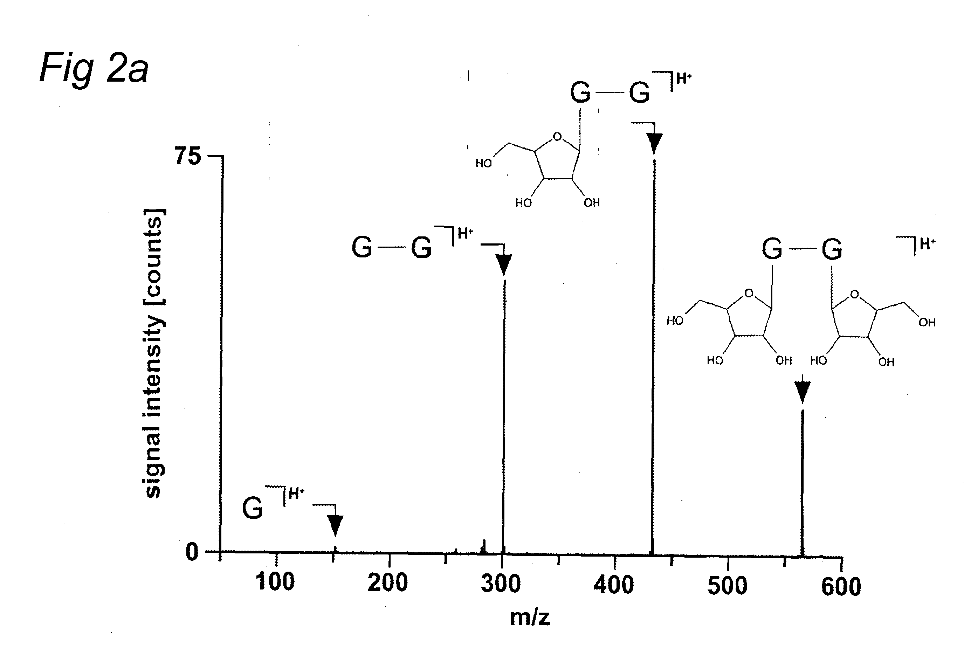

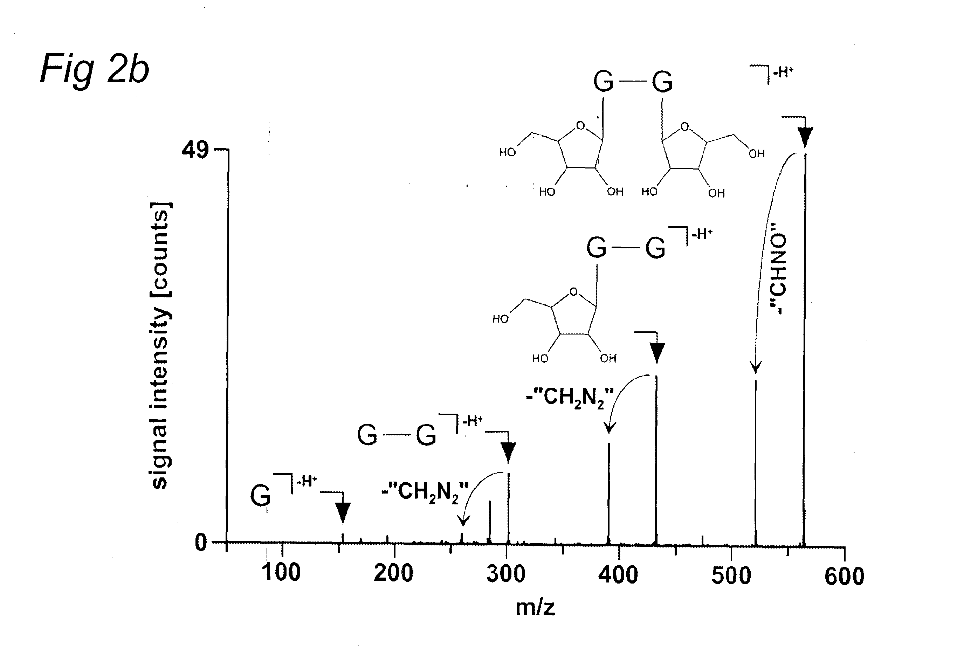

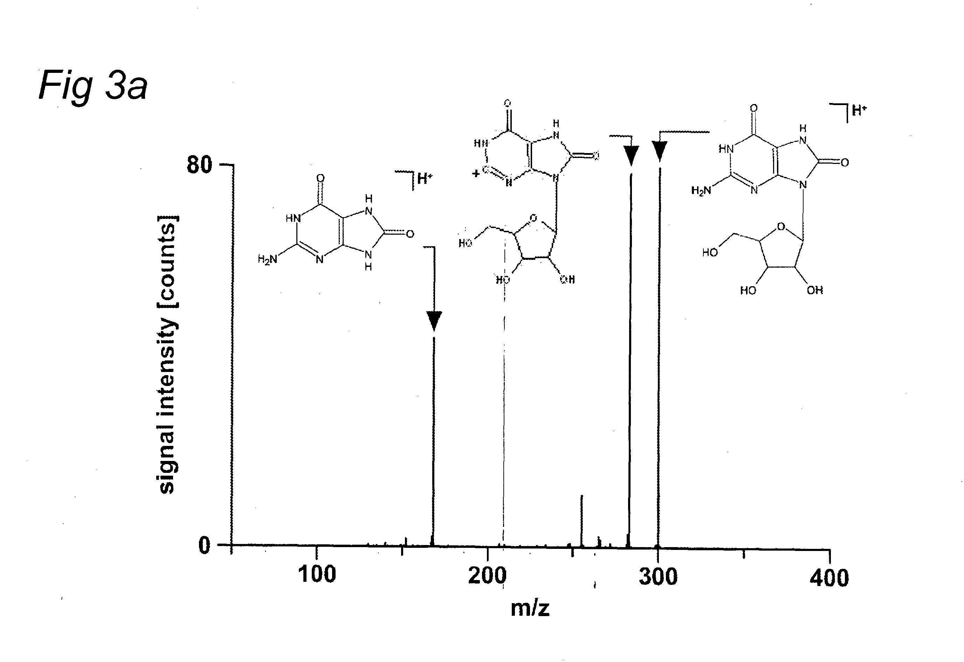

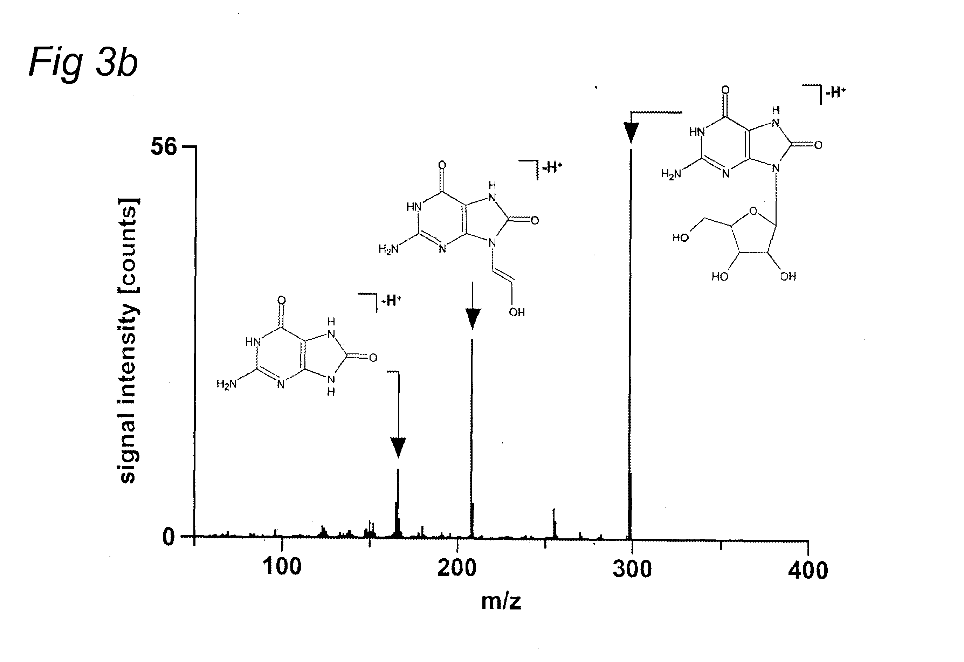

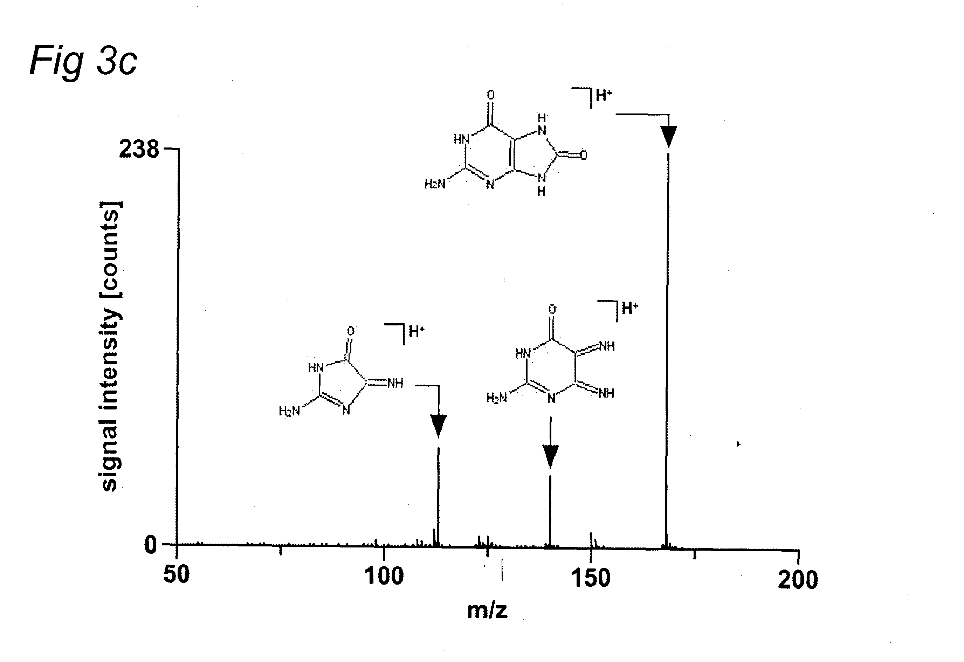

In Figure 2 fragment ion mass spectra obtained from MS/MS of both the protonated and deprotonated guanosine dimer are depicted. In both spectra consecutive dissociation of the riboses from the dimeric species was observed clearly indicating that the two guanosines were linked with each other via the nucleobases. In Figure 3 fragment ion mass spectra obtained from monooxidized guanosine are shown. The most abundant signals in the MS/MS spectrum of the protonated form originated from deamination and cleavage of the ribose. The fragmentation behavior of the deprotonated form is distinctly different. Besides the complete loss of the sugar giving rise to the formation of the deprotonated nucleobase, a specific loss of "C₃H₆O₃" induced by the cleavage of C-C and C-O bonds of the sugar ring was observed, which represented a unique identifier for the formation of 8-hydroxyguanosine.

Only for a limited number of oxidation products the obtained MS/MS data was specific enough to unequivocally elucidate the structure of the modified nucleobase. This problem was solved by combining in-source fragmentation with MS/MS. The protonated nucleobase was cleaved from the ribose by in-source fragmentation; specific fragment ions of the nucleobase were obtained by MS/MS. In Figure 3c the fragment ion mass spectrum obtained from the protonated form of the monooxidized nucleobase is shown. The spectrum resembles to a large extend published spectra obtained from the fragmentation of 8-hydroxyguanine (Malayappan et al. J Chromatogr A 2007, 1167, 54-62), which proves that 8-hydroxyguanosine represents the monooxidized form of guanosine.

These data show that EC/ESI-MS represents a valuable tool for studying the fundamental principles of nucleobase oxidation and that this can advantageously be used to gain a better understanding of redox reactions involving nucleic acids in biological systems.

### Example 3

### Electrochemical oxidation of nucleoticles.

The usefulness of EC/ESI-MS for studying the electrochemical oxidation of guanosine monophosphat (GMP) and of small homooligomers (dGpdG and dGpdGpdG) was studied.

The following sample solutions were used:
(a) 54 µM guanosine monophosphat in 20 mM ammonium formate (pH 7.3) containing 50% acetonitrile (v/v);
(b) 28 µM dGpdG in 10 mM cyclohexyldimethylammonium acetate (CycHDMAA, pH 8.3) containing 20% methanol (v/v);
(c) 28 µM dGpdGpdG in 10 mM CycHDMAA (pH 8.3) containing 20% methanol (v/v).

ESI-MS was performed in negative mode. Accurate molecular mass measurements and tandem mass spectrometric experiments were used to characterize the oxidation products. Potentials were ramped from 0.0 V to 3.0 V to induce oxidation. Oxidation products were detected at voltages beyond 1.5 V.

For GMP, conformity with the mechanism postulated for electrochemical oxidation of guanosine was observed. In Figure 4 the MS/MS spectrum of the monooxidized form of GMP is shown. The fragment ion mass spectrum exhibited a number of characteristic fragment ions of GMP. Signals corresponding to "PO₃⁻", "H₂PO₄⁻", as well as to the cleaved ribose-5'-phosphate represented unique identifiers.

The tandem mass spectrum of 8-hydroxy-GMP is of particular interest for studying the fragmentation behavior of different species containing 8-hydroxyguanine. For 8-hydroxyguanosine, the most abundant signal upon fragmentation of the deprotonated molecular ion resulted from internal cleavage within the ribose (Figure 3). The proposed mechanism includes cleavage of C-C and C-O bonds within the sugar ring with the charge localized to the sugar as a deprotonated hydroxyl group. Upon collision induced decomposition of monooxidized GMP, internal fragmentation of the ribose occurred. However, the corresponding fragmentation pathway was disfavoured. Most probably due to the localization of the negative charge at the phosphate group, the release of the deprotonated nucleobase represented the preferred fragmentation reaction.

For dGpdG mono- and doubly-oxidized species were detected. MS/MS experiments were performed to elucidate the structure of these modified forms. The experiments clearly revealed the coexistence of different structural isomers. Isomers exhibiting identical molecular masses were differentiated by characteristic fragment ions. The tandem mass spectrum obtained form fragmentation of the monooxidized forms of dGpdG is shown in Figure 5a. The occurrence of w1 ions containing either guanine or its oxidized form indicates that both nucleobases can be oxidized within the dimer. The signal intensity difference of the w₁ ions suggests that oxidation of the 5'-terminal guanine might be favored. However, since the oxidized form seems to be preferentially cleaved from the backbone, it is difficult to decide if one of the two guanosines is really preferentially oxidized. For the doubly-oxidized form, three different w₁ ions containing either guanine, its oxidized form, or its doubly oxidized form were observed in the MS/MS spectrum (Figure 5b) which clearly suggests the occurrence of three different isomers. In one species both guanines were mono-oxidized; in the two other species a single guanine residue was doubly-oxidized. Obviously, consecutive oxidation of an oligomer can either affect different sites or can just occur at a single guanine residues (Figure 5c).

For dGpdGpdG a mixture of different mono-oxidized species was detected. The guanines were identified as the sites of modification; no clear evidence for the preferred oxidation of any nucleotide position was observed.

### Example 4

### Formation of drug-DNA adducts.

The usability of EC/ESI-MS to study the reactivity of small molecules with nucleic acids was evaluated.

For this purpose the formation of a covalent adduct between guanosine and acetaminophen was studied (paracetamol, N-acetyl-p-aminophenol, APAP, CAS No. 103-90-2).

Acetaminophen is a widely used over-the-counter analgesic and antipyretic, and is therefore a major ingredient in numerous cold and flu remedies. Several studies on cell cultures and rodents have demonstrated that acetaminophen can covalently bind to nucleic acids after metabolic activation. The structure of the formed DNA adduct(s) has not been elucidated yet.

In this experiment EC was used to produce reactive intermediates. A mixture of acetaminophen and guanosine (50 µM acetaminophen and 320 µM guanosine in 10 mM ammonium formate (pH 7.3) containing 30% acetonitrile (v/v)) was passed through the electrochemical cell described herein before and monitored with ESI-MS. The potential was ramped from 0.0 V to 3.0 V. Oxidation of APAP was observed at voltages beyond 1.2 V. Even at 3.0 V the signal intensity does not fall.below 90% of its initial value. Guanosine was stable up to 1.6 V. Higher potentials induced guanosine oxidation. Interestingly, even at a potential of 3.0 V, which would have been high enough to completely oxidize guanosine in a pure solution, the normalized intensity does not fall below 50%. This finding seems to suggest that APAP has antioxidant properties.

The oxidation of APAP is believed to be induced by the loss of one electron and one proton, resulting in an intermediate radical. Radical formation is indicated by APAP dimer formation. Extensive APAP dimer formation was observed at 1.2-1.6 V. Adduct formation did only occur after the electrochemical activation of guanosine. Activation of guanosine was indicated by the formation of guanosine oxidation products such as guanosine dimer. The first step of guanosine oxidation is the loss of one electron and one proton, resulting in an intermediate radical. This radical can react with different species including itself and the APAP radical.

As is evident from these results, adduct formation was only observed at potentials sufficiently high to generate guanosine radicals.

Accurate molecular measurements and tandem mass spectrometric experiments were used to prove the adduct formation. Observed fragmentation pathways included the loss of "NH₃" and of the ribose, which are typical for guanosine, and the loss of acetamide, which is a known fragmentation reaction of acetaminophen.

The generally accepted mechanism of adduct formation includes activation of adduct-forming agent to a reactive electrophile that can bind to nucleophilic sites of nucleic acids. The above results clearly suggest that coactivation of the nucleobase significantly increases the risk of adduct formation.

These findings show that the present method may suitably be used to study the impact of chemical structure and functional groups on the reactivity of molecules with nucleic acids. Such studies may help to increase the accuracy of *in silico* methods that are used to predict a chemical's mutagenic activity. Furthermore, such studies may yield a better understanding of the reaction mechanisms that lead to the formation of adducts of nucleic acids. Thus, the present method may be used to facilitate the design of new potent antitumor drugs, as such drugs ideally exhibit high reactivity with nucleic acids.

A major benefit of the present assay resides in the exclusion of unwanted side reactions with other biological nucleophiles. Standard procedures for bioactivation, such as enzymatic incubations or *in vivo* experiments, yield only small amounts of modified nucleobases, which have to be isolated from a complex biological matrix for identification and characterization, while EC/ESI-MS yields very clean modified nucleic acids directly amenable to mass-spectrometric analysis. This saves time, labour, and money, and reduces the probability of erroneous results due to problems related to sample preparation.

### Example 5

### On-line EC/LC/MS of guanosine - Identification of reaction products

On-line coupling of Electrochemistry (EC) with Liquid Chromatography (LC) and Mass Spectrometric (MS) detection enables the differentiation of isomeric and isobaric compounds, the analysis of multi-component samples and the analysis of compounds in complex matrices. Low flow rates through the electrochemical reactor cell are preferred to secure maximum electrochemical conversion. The volume of the electrochemical reactor cell and the used flow rate through the cell should be tailored to the chromatographic separation system (LC) to obtain best performance regarding sampling times, run times and detection limits.

A sample solution of 100 µM guanosine dissolved in 10 mM ammonium formate (pH 7.3) was subjected to electrochemical simulation followed by on-line chromatographic separation using a ROXY™ EC/LC system (Antec, Zoeterwoude, The Netherlands), which had been modified to allow operation at low flow rates, i.e., capillary LC was used. The EC/LC system was used in conjunction with a mass spectrometer (QSTAR™ XL: Applied Biosystems, Foster City, CA) for detection.

The potential applied in the EC was 1750 mV. The flow rate employed in the EC was 3 µl/min. Otherwise the experimental conditions were identical to those applied in the EC/MS analysis of guanosine described in Example 2.

### EC/LC conditions:

Column:
   - 200×0.2 mm i.d., Eurospher™ 100-5 C18 (Knauer, Berlin, Germany)
Mobile phase:
   - A: 10 mM ammonium formate (pH 7.3)
   - B: 10 mM ammonium formate (pH 7.3) containing 50% acetonitrile
   - Gradient: 5-30% B in 10 min

A primary flow of 150 µl/min was split to 2.0 µl/min with the help of a T-piece and a 50 µm i.d. fused-silica restriction capillary. The i.d. of the connecting capillaries was 20-25 µm. The injection volume was 2.0 µl.

### Results:

Using on-line EC/LC/MS allowed for the separation and unambiguous identification of all relevant components (educt and oxidation/reaction products). More particularly the findings were as follows:
(1) Same reaction products as in EC/MS experiment.
(2) Only the dimer and 8-hydroxyguanosine were retained on the column.
(3) A new species was found at m/z of 553.176.
(4) The sample solution contained guanine. The guanine is either an impurity from the production process or is a degradation product of guanosine.

### Example 6

### EC/LC/MS of guanosine - Mass voltammogram:

Using the same conditions as described in Example 5, a mass voltammogram was produced by operating the EC at different potentials in the range of 0-3000 mV.

It was found that disturbing ion suppression effects, which are usually observed in on-line EC/MS and which can severely hamper quantitative experiments, were prevented or at least suppressed by the chromatographic separation prior to mass spectrometric detection.

In addition, the following observations were made:
(1) At voltages higher than 1000 mV guanosine starts to decrease due to oxidation. Further increase in voltage results in enhanced oxidation of guanosine. At 1500 mV less than half of the original signal remains indicating a 50% oxidation.
(2) At potentials higher than 1250 mV 8-hydroxyguanosine is formed. The amount increases with increasing potential.
(3) At potentials higher than 1000 mV guanosine dimer was formed. The amount of dimer formed reaches its maximum at approximately 1250-1500 mV and decreases to zero at 2000 mV.
(4) The chromatogram showed that between 1000 and 2000 mV two different dimers are formed (retention times of 11.3 and 14.3 min.). The voltammograms of the guanosine dimers differed, illustrating for the first time ever the additional selectivity gain obtained by the electrochemical reaction process in an on-line EC/LC/MS set-up.
(5) The measured mass voltammogramms provide valuable insights into the mechanism of electrochemical guanosine oxidation. The first step of guanosine oxidation is the loss of one electron and one proton, resulting in an intermediate radical. This radical reacts with another radical to form dimers. At higher potentials it seems as if the primarily formed dimers are oxidized to 8-hydroxyguanosine. This has been confirmed recently by: Mautjana, N. A.; Looi, D. W.; Eyler, J. R.; Brajter-Toth, Electrochim. Acta 2009, 55, 52-58.

### Example 7

### EC/LC/MS of guanosine - Conversion efficiency

The conversion efficiency of the EC/LC/MS system described in Example 5 was tested by employing flow rates of 0.5-15 µl/min.

It was found that best performance is obtained at flow rates between 2-5 µl/min. At higher flow rates the conversion efficiency is lower. At lower flow rates residence times become so high that the primary oxidation products are converted. In other words, at flow rates well below 2 µl/min secondary oxidation reactions occur that significantly reduce the amount of the educt as well as of the primary oxidation products (dimers, 8-hydroxyguanosine).

### Example 8

### EC/LC/MS/MS of a mixture of guanosine and Acetaminophen

The set-up and conditions used in this experiment were identical to those described in Example 5, except that:
- tandem mass spectrometry was used for detection (Precursor ion: 433.1; collision energy: 15 eV)
- the samples contained a mixture of guanosine and Acetaminophen (350 µM guanosine and 200 µM Acetaminophen in 10 mM ammonium formate (pH 7.3))
- a different gradient was used (5-60% B in 10 min.)

On-line EC/LC/MS/MS (precursor m/z = 433.1) resulted in the detection of 4 adducts, with different fragment ion mass spectra. These adducts eluted at 9.3 min., 9.7 min., 10.1 min. and 11.5 min. Also detected was an unknown compound that eluted at 10.8 min.

### Example 9

### EC/LC/MS/MS of a mixture of guanosine and Acetaminophen - Impact of the Acetaminophen concentrations on the amount of produced adduct

The experiment described in Example 8 was repeated, except that the Acetaminophen concentration in the sample was varied from 0-350 µM. The effect of the Acetaminophen concentrations on the amounts of adduct formed could be determined easily from the data generated in this experiment. The amount of guanosine-Acetaminophen adduct formed was found to increases with increasing concentration of Acetaminophen.

The unknown adduct mentioned in Example 8 (retention time = 10.8 min.) was detected even when no Acetaminophen was present in the sample solution and its concentration remained nearly constant irrespective of the added amount of Acetaminophen.

Curves such as the ones produced in this experiment can be used to compare the reactivity of adduct forming species and represent a tool for assessing the "risk of adduct formation".

### Example 10

### EC/LC/MS/MS of a mixture of guanosine and acetaminophen - Mass voltammograms

The experiment described in Example 8 was repeated, except that this time a mass voltammogram was produced by operating the EC at different potentials in the range of 0-4000 mV. Furthermore, tandem mass spectrometry was performed on precursor ions 433.1, 300.1, 301.1 and 565.1.

Two different Acetaminophen dimers were identified (dimer 1 with retention time 11.3 min and dimer 2 with retention time 14.0 min).

Furthermore, the following findings were made::
(1) Guanosine oxidation started at around 1250 mV
(2) The voltammograms of the guanosine dimers were different.
(3) Maximum concentration for one guanosine dimer was reached at appr. 1800 mV and for the other dimer at appr. 2500 mV.
(4) The amount of 8-hydroxyguanosine increased till appr. 3500 mV and sharply decreased when the potential was increased further.
(5) Maximum concentrations for the two Acetaminophen dimers were reached at approx. 1000 mV.
(6) The voltammograms of the 4 different guanosine-Acetaminophen adducts were quite similar.
(7) Maximum concentrations for the four guanosine-Acetaminophen adducts were reached at appr. 1800 mV.
(8) Guanosine oxidation is mandatory for adduct formation.

## Claims

1. A method of determining the impact of an agent on oxidation reactions of a nucleic acid species selected from nucleosides, nucleotides, oligonucleotides, polynucleotides and combinations thereof, said method comprising the steps of:
• providing a reference sample containing a nucleic acid species;
• providing a test sample containing the same nucleic acid species as the reference sample;
• oxidizing the reference sample and the test sample by submitting each sample to identical electrochemical oxidation conditions, said oxidation conditions being sufficiently severe to produce detectable levels of oxidation products of the nucleic species in the reference sample;
• on-line determining the oxidation profile of the electrochemically oxidized reference sample and of the electrochemically oxidized test sample using Electrospray Ionization-Mass Spectrometry (ESI-MS) or Matrix Assisted Laser Desorption Ionization-Mass Spectrometry (MALDI-MS); and
• determining the impact of the agent on oxidation reactions of nucleic acid species by comparing the oxidation profiles of the reference sample and the test sample;
wherein the agent is added to the test sample before the on-line determining of the oxidation profile.

2. Method according to claim 1, wherein the comparison of the oxidation profiles of the reference sample and the test sample comprises quantitative comparison of the recorded intensity at one or more predetermined mass-to-charge (m/z) ratios for each sample, said m/z ratios coinciding with the m/z ratios of one or more oxidation products of nucleic acid species.

3. Method according to claim 1, wherein the comparison of the oxidation profiles of the reference sample and the test sample comprises identifying m/z ratios having a statistically significant higher intensity in the oxidation profile of the test sample than in the oxidation profile of the reference sample and determining whether these m/z ratios are associated with a potential adduct of the agent and an oxidation product of the nucleic acid species.

4. Method according to claim 1, wherein the on-line determining of the oxidation profile comprises feeding at least a part of the electrochemically oxidized reference sample and at least a part of the electrochemically oxidized test sample to a chemical separation device that is coupled to the ESI-MS or the MALDI-MS, using identical separation conditions for the oxidized samples.

5. Method according to claim 4, wherein the chemical separation device is a liquid chromatograph.

6. Method according to claim 4, wherein both the reference sample and the test sample are oxidized in a Flow-through Electrochemical Cell (FEC) that is coupled to the chemical separation device, said electrochemical cell having a working volume of 1.0 nl - 1.0 ml and being operated at a flow rate of 1.0 nl/min - 10 ml/min.

7. Method according to claim 6, wherein 1.0 nl -1.0 ml of oxidized eluate from the FEC is collected before being introduced into the chemical separation device for on-line determining of the oxidation profile.

8. Method according to claim 6, wherein the reference sample and the test sample are oxidized in the same electrochemical cell.

9. Method according to claim 1, wherein the same biological material is present in the reference sample and the test sample, said biological material being selected from a bodily fluid, tissue or a material derived from bodily fluid or tissue.

10. Method according to claim 1, wherein the agent is added to test sample before the test sample is subjected to electrochemical oxidation conditions.

11. Method according to claim 1, wherein the test sample is provided by an autosampler.

12. Method according to claim 11, wherein the method comprises on-line determining the oxidation profiles of at least 10 test samples in a single run of the autosampler.

13. Method according to claim 1, wherein method comprises on-line determining of at least 2 oxidation profiles of electrochemically oxidized samples per hour.

14. Method according to claim 13, wherein the method comprises on-line determining of at least 10 oxidation profiles of electrochemically oxidized test samples per hour and wherein the oxidized samples are directly transferred to the ESI-MS or the MALDI-MS without prior chromatographic separation.

15. Method according to claim 1, wherein the nucleic acid species is selected from nucleotides, oligonucleotides and combinations thereof.

## Patentansprüche

1. Ein Verfahren zur Bestimmung des Einflusses eines Stoffs auf Oxidationsreaktionen einer Nukleinsäurespezies, ausgewählt aus Nucleosiden, Nukleotiden, Oligonukleotiden, Polynukleotiden und deren Gemischen, umfassend die Schritte:
• Bereitstellen einer Vergleichsprobe, enthaltend eine Nukleinsäurespezies,
• Bereitstellen einer Versuchsprobe, enthaltend die gleiche Nukleinsäurespezies wie die Vergleichsprobe,
• Oxidation der Vergleichsprobe und der Versuchsprobe durch Aussetzen jeder Probe identischen elektrochemischen Oxidationsbedingungen, wobei diese Oxidationsbedingungen ausreichend stark sind, um nachweisbare Spiegel von Oxidationsprodukten der Nukleinsäurespezies in der Vergleichsprobe zu erzeugen;
• Online-Ermittlung der Oxidationsprofile der elektrochemisch oxidierten Vergleichsprobe und der elektrochemisch oxidierten Versuchsprobe durch Verwendung von Elektrospray-Ionisations-Massenspektrometrie (ESI-MS) oder Matrix-unterstützter Laser Desorptions/Ionisations-Massenspektrometrie (MALDI-MS) und
• Ermittlung des Einflusses eines Stoffs auf die Oxidationsreaktionen einer Nukleinsäurespezies durch Vergleich der Oxidationsprofile der Vergleichsprobe und der Versuchsprobe,
wobei die Zugabe des Stoffs zur Versuchsprobe vor der Online Ermittlung des Oxidationsprofils erfolgt.

2. Verfahren nach Anspruch 1, worin der Vergleich der Oxidationsprofile der Vergleichsprobe und der Versuchsprobe den quantitativen Vergleich der aufgenommenen Intensität an einem oder mehr vorbestimmten Masse zu Ladungs (m/z)-Verhältnissen für jede Probe umfasst, wobei diese m/z-Verhältnisse mit den m/z-Verhältnissen von einem oder mehr Oxidationsprodukten der Nukleinsäurespezies übereinstimmen.

3. Verfahren nach Anspruch 1, worin der Vergleich der Oxidationsprofile der Vergleichsprobe und der Versuchsprobe die Identifikation von m/z-Verhältnissen mit einer statistisch signifikant höheren Intensität des Oxidationsprofils des Versuchsbeispiels gegenüber dem Oxidationsprofil des Vergleichsbeispiels und die Ermittlung, ob diese m/z-Verhältnisse mit einem potentiellen Addukt des Stoffs und einem Oxidationsprodukt der Nukleinsäurespezies übereinstimmen, umfasst.

4. Verfahren nach Anspruch 1, worin die online-Ermittlung des Oxidationsprofils die Zuführung von mindestens einem Teil der elektrochemisch oxidierten Referenzprobe und mindestens einem Teil der elektrochemisch oxidierten Versuchsprobe in ein Gerät zur chemischen Trennung, das mit dem ESI-MS oder dem MALDI-MS gekoppelt ist, unter Verwendung von identischen Trennungsbedingungen für die oxidierten Proben, umfasst.

5. Verfahren nach Anspruch 4, worin das Gerät zur chemischen Trennung ein Flüssigchromatograph ist.

6. Verfahren nach Anspruch 4, worin sowohl die Vergleichsprobe als auch die Versuchsprobe in einer elektrochemischen Durchflusszelle (FEC) oxidiert werden, die an das Gerät zur chemischen Trennung gekoppelt ist, wobei diese elektrochemische Zelle ein Arbeitsvolumen von 1,0 nl bis 1,0 ml hat und bei einer Fließrate von 1,0 nl/min bis 10 ml/min betrieben wird.

7. Verfahren nach Anspruch 6, worin 1,0 nl bis 1,0 ml des oxidierten Eluats der FEC gesammelt werden, bevor sie in das Gerät zur chemischen Trennung zur Online-Ermittlung der Oxidationsprofile eingeführt werden.

8. Verfahren nach Anspruch 6, worin die Vergleichsprobe und die Versuchsprobe in der gleichen elektrochemischen Zelle oxidiert werden.

9. Verfahren nach Anspruch 1, worin das gleiche biologische Material in Vergleichsprobe und Versuchsprobe vorhanden ist, dieses biologische Material ist ausgewählt aus einer Körperflüssigkeit, Gewebe oder einem Material, das aus Körperflüssigkeit oder Gewebe stammt.

10. Verfahren nach Anspruch 1, worin der Stoff zur Versuchsprobe gegeben wird, bevor die Versuchsprobe elektrochemischen Oxidationsbedingungen ausgesetzt wird.

11. Verfahren nach Anspruch 1, worin die Versuchsprobe durch einen Autosampler bereitgestellt wird.

12. Verfahren nach Anspruch 11, worin das Verfahren die Online-Ermittlung der Oxidationsprofile von mindestens 10 Versuchsproben in einem einzigen Durchlauf des Autosamplers umfasst.

13. Verfahren nach Anspruch 1,worin das Verfahren die Online-Ermittlung von mindestens 2 Oxidationsprofilen von elektrochemisch oxidierten Proben pro Stunde umfasst.

14. Verfahren nach Anspruch 13, worin das Verfahren die Online-Ermittlung von mindestens 10 Oxidationsprofilen von elektrochemisch oxidierten Versuchsproben pro Stunde umfasst und wobei die oxidierten Proben ohne vorhergehende chromatographische Trennung direkt zum ESI-MS oder MALDI-MS überführt werden.

15. Verfahren nach Anspruch 1, worin die Nukleinsäurespezies ausgewählt ist aus Nukleotiden, Oligonukleotiden und deren Gemischen.

## Revendications

1. Procédé de détermination de l'impact d'un agent sur des réactions d'oxydation d'une espèce d'acide nucléique sélectionnée parmi des nucléosides, nucléotides, oligonucléotides, polynucléotides et des combinaisons de ceux-ci, le procédé comprenant les étapes consistant à :
- fournir un échantillon de référence contenant une espèce d'acide nucléique ;
- fournir un échantillon test contenant la même espèce d'acide nucléique que l'échantillon de référence ;
- oxyder l'échantillon de référence et l'échantillon test en soumettant chaque échantillon à des conditions d'oxydation électrochimique identiques, les conditions d'oxydation étant suffisamment sévères pour produire des niveaux détectables de produits d'oxydation de l'espèce nucléique dans l'échantillon de référence ;
- déterminer en ligne le profil d'oxydation de l'échantillon de référence oxydé électrochimiquement et de l'échantillon test oxydé électrochimiquement en utilisant une spectrométrie de masse avec ionisation par pulvérisations d'électrons (ESI - MS) ou une spectrométrie de masse avec désorption-ionisation laser assistée par matrice (MALD 1 - M S) et
- déterminer l'impact de l'agent sur les réactions d'oxydation d'une espèce d'acide nucléique en comparant les profils d'oxydation de l'échantillon de référence et de l'échantillon test ;
dans lequel l'agent est ajouté à l'échantillon test avant la détermination en ligne du profil d'oxydation.

2. Procédé selon la revendication 1, dans lequel la comparaison des profils d'oxydation de l'échantillon de référence et de l'échantillon test comprend une comparaison quantitative de l'intensité enregistrée au niveau d'un ou plusieurs rapports masse sur charge (m/z) prédéterminés pour chaque échantillon, les rapports m/z coïncidant avec les rapports m/z d'un ou plusieurs produits d'oxydation d'une espèce d'acide nucléique.

3. Procédé selon la revendication 1, dans lequel la comparaison des profils d'oxydation de l'échantillon de référence et de l'échantillon test consiste à identifier des rapports m/z ayant une intensité statistiquement significative plus élevée dans le profil d'oxydation de l'échantillon test que dans le profil d'oxydation de l'échantillon de référence et déterminer si ces rapports m/z sont associés à un produit d'addition potentiel de l'agent et d'un produit d'oxydation de l'espèce d'acide nucléique.

4. Procédé selon la revendication 1, dans lequel la détermination en ligne du profil d'oxydation consiste à fournir au moins une partie de l'échantillon de référence oxydé électrochimiquement et au moins une partie de l'échantillon test oxydé électrochimiquement à un dispositif de séparation chimique qui est relié à la ESI - MS ou la MALD 1 - M S, en utilisant des conditions de séparation identiques pour les échantillons oxydés.

5. Procédé selon la revendication 4, dans lequel le dispositif de séparation chimique est un chromatographe liquide.

6. Procédé selon la revendication 4, dans lequel à la fois l'échantillon de référence et l'échantillon test sont oxydés dans une cellule électrochimique à flux traversant (FEC) qui est reliée au dispositif de séparation chimique, la cellule électrochimique ayant un volume actif de 1,0 ni à 1,0 ml et étant activée à un débit de 1,09 nl/min à 10 ml/min.

7. Procédé selon la revendication 6, dans lequel 1,0 ni à 1,0 ml d'éluat oxydé provenant de la FEC est recueilli avant d'être introduit dans le dispositif de séparation chimique pour détermination en ligne du profil d'oxydation.

8. Procédé selon la revendication 6, dans lequel l'échantillon de référence et l'échantillon test sont oxydés dans la même cellule électrochimique.

9. Procédé selon la revendication 1, dans lequel le même matériau biologique est présent dans l'échantillon de référence et l'échantillon test, le matériau biologique étant sélectionné parmi un fluide corporel, un tissu ou un matériau dérivé d'un fluide corporel ou d'un tissu.

10. Procédé selon la revendication 1, dans lequel l'agent est ajouté à l'échantillon test avant que l'échantillon test ne soit soumis à des conditions d'oxydation électrochimique.

11. Procédé selon la revendication 1, dans lequel l'échantillon test est fourni par un auto-échantillonneur.

12. Procédé selon la revendication 11, dans lequel le procédé consiste à déterminer en ligne les profils d'oxydation d'au moins 10 échantillons test dans un seul fonctionnement de l'auto-échantillonneur.

13. Procédé selon la revendication 1, dans lequel le procédé consiste à déterminer en ligne au moins 2 profils d'oxydation d'échantillons oxydés électrochimiquement par heure.

14. Procédé selon la revendication 13, dans lequel le procédé consiste à déterminer en ligne au moins 10 profils d'oxydation d'échantillons test oxydés électrochimiquement par heure et dans lequel les échantillons oxydés sont directement transférés vers la ESI - MS ou la MALD I - M S sans séparation chromatographique préalable.

15. Procédé selon la revendication 1, dans lequel l'espèce d'acide nucléique est sélectionnée parmi des nucléotides, oligonucléotides et des combinaisons de ceux-ci.
